# EUROPEAN PATENT APPLICATION

(11) **EP 2 033 620 A1**
(43) Date of publication of application: **11.03.2009**
(21) Application number: 07788547.3
(22) Date of filing: 18.05.2007
(51) Int. Cl.: A61K 8/14, A61K 9/127, A61K 31/05, A61K 31/216

(54) **FORMULATION OF NATURAL TRITERPENES AND BIOPHENOLS OBTAINED FROM THE GENUS OLEA IN LIPOSOMES**

(30) Priority: 18.05.2006 ES 200601435
(71) Applicant: Universidad de Granada, 18071 Granada (ES)
(72) Inventor: GARCIA-GRANADOS LOPEZ DE HIERRO, Andres, E-18071 Granada (ES)
(74) Representative: Carpintero Lopez, Francisco
(86) International application number: PCT/ES2007/000288
(87) International publication number: WO 2007/135210

(57) **Abstract**

The present invention relates to compositions of natural biophenols and, optionally, of natural biophenols and triterpenes obtained from the genus Olea. The compositions comprise said biophenols and/or triterpenes included in liposomes. The preferred biophenols within the context of the invention are tyrosol and hydroxytyrosol and the preferred triterpenes are maslinic acid and oleanolic acid and/or their salts.

## Description

### OBJECT OF THE INVENTION

The present invention consists of a formulation of natural triterpenes and biophenols obtained from the genus Olea in liposomes, specifically of triterpenes from the group comprised of maslinic acid and/or its salts, oleanolic acid and/or its salts, tyrosol and hydroxytyrosol in liposomes.

### STATE OF THE ART

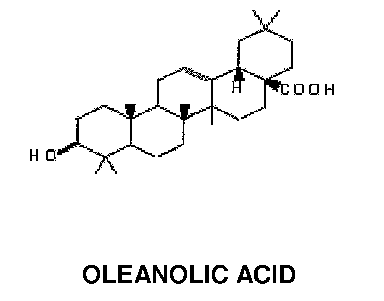

Oleanolic acid (3-beta-hydroxy-28-carboxy-oleanene) is a triterpenic acid ubiquitously distributed within the plant kingdom. Thus, the phytochemical database of the United States Department of Agriculture (Internet Address http://www.arsgrin.gov:8080/npgspub/xsql/duke/chemdisp.xsql?chemical=OLEANOLIC-ACID) is present in 130 plants, one of which is Olea europaea, and moreover a number of proven biological activities (abortifacient, anti-cariogenic, antifertility, antihepatotoxic, anti-inflammatory, anti-sarcomic, cancer-preventive, cardiotonic, diuretic, hepatoprotective, uterotonic, etc., up to 38 different properties). Numerous studies have been published on the possible biological activity of this acid and its glucosides. It has been studied in its activity as an inhibitor of the proliferation of leukaemia cells (Essady, D., Najid, A., Simo, A., Denizot, Y., Chulia, A.J. and Delage, C.; Mediators of Inflammation (1994) 3, 181-184), as a hypoglycemiant (Yoshikawa,M., Matsuda,H., Harada,E., Mukarami, T., Wariishi, N., Murakami,N. And Yamahara,J.; Chemical & Pharmaceutical Bulletin, (1994) 42, 1354-1356) an antitumoral (Ohigashi,H., Mukarami,A. and Koshimizu,K ACS Symposium Series (1994) 547, 251-261), a producer of antagonistic effects in anaphylactic shock (Zhang,L.R. and Ma, T.X.; Acta Pharmacológica Sinica (1995)16, 527-530), hepatoprotective (Liu,J., Liu,Y.P., Parkinson,A. and Klaasen, C.D. ; Journal of Pharmacology and Experimental Therapeutics, (1995) 275, 768-774 ; Connolly, J.D. and Hill, R.A.Natural Product Reports 12, 609-638 (1995), anti-inflammatory (Recio,M.D., Giner,R.M., Manez,S. And Rios, J.L. ; Planta Medica (1995) 61, 182-185. A specific review of the pharmacological activity of oleanolic acid has been published (Liu, J. Journal of Ethnopharmacology (1995) 49, 57-68).

Other derivatives of oleanolic acid, in addition to echinocystic acid (16-hydroxy-oleanolic) have been found to have inhibiting effects against the replication of HIV in H-9 cells with EC50 values of 2.3 mM (Anti-AIDS agents, 21. Triterpenoid saponins as anti-HIV principles from fruits of Gleditsia japonica and Gymnocladus chinensis, and a structure-activity correlation, Konoshima, Takao; Yasuda, Ichiro; Kashiwada, Yoshiki; Cosentino, L. Mark; Lee,Kuo-Hsiung, J. Nat. Prod., 58(9), 1372-7, (1995)). Other many direct derivatives have been found to be Leukotriene D4 antagonists (Leukotriene D4 antagonists in Tripterygium wilfordii, Morota, Takashi; Saitoh, Kazuko; Maruno, Masao; Yang, Chun-Xin; Qin, Wan-Zhang; Yang, Bing-Hui, Nat. Med., 49(4), 468-71 (1995)).

The best proof of the interest that it arouses worldwide is the number of international patents that exist in relation to this acid: Use of oleanolic acid as a vasodilator and restorer agent for endothelial dysfunction (W02004ES00190 20040430); Cosmetic and dermopharmaceutical compositions for skin prone to acne (WO2002FR03344 20021001); Cosmetic composition for care of sensitive skin includes oleanolic acid or vegetable extract rich in oleanolic acid, and at least one other vegetable extract chosen from shea-butter flower and solanum lycocarpum (FR20000008758 20000705); Process for preparing food products fortified with oleanolic acid (US19990468637 19991222); Oleanolic acid-based anti-pruritus agent (JP19970183075 19970623); Angiogenesis inhibitor composition comprising oleanolic acid (KR19920021117 19921111).

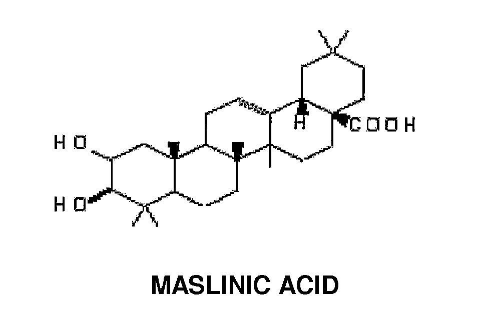

Maslinic acid (2-alpha, 3-beta-dihydroxy-28-carboxy-oleanene), also known as crataegolic acid, is far less widespread in nature, and has been found in 23 plants (Internet Address http://www.arsgrin.gov:8080/npgspub/xsql/duke/chemdisp.xsql?chemical=MASLINIC-ACID). It is known to have antihistaminic and anti-inflammatory properties (Internet Address http://www.arsgrin.gov:8080/npgspub/xsql/duke/chemdisp.xsql?chemical=MASLINIC-ACID) although it has not been extensively studied because of its scarcity. The isolation of oleanolic and maslinic acids from waxes on the surface of the fruit of Olea europaea has been described (Bianchi,G., Pozzi,N. And Vlahov, G. Phytochemistry (1994) 37, 205-207) by means of methanol extraction from olives previously washed with chloroform. The separation of acids of this type has been described by means of high-speed counter-flow chromatography (HSCCC)( Du, Q.Z., Xiong, X.P. and Ito, Y. ; Journal of Liquid Chromatography (1995) 18, 1997-2004.

Maslinic acid has recently been found to have a powerful inhibiting activity in vitro of the Aids virus protease (HIV-1) (Anti-HIV Triterpene Acids from Geum japonicum, Xu, H.X.; Zeng, F.; Wan, M.; Sim, Keng-Yeow J. Nat. Prod., 59(7), 643-645 (1996)). It is also a product of the future, as revealed by a pharmacophore search for HIV-1 protease inhibitors, performed by the National Cancer Institute (Betsheda, USA), which has pinpointed a by-product of maslinic acid as a promising basis for future development in this activity (Discovery of Novel, Non-Peptide HIV-1 Protease Inhibitors by Pharmacophore Searching, Wang, Shaomeng; Milne, G. W. A.; Yan, Xinjian; Posey, Isadora; Nicklaus, Marc C.; GrahamX Lisa; Rice, William G., J. Med. Chem., 39(10), 2047-54 (1996)).

As a result of the biological tests performed by the University of Granada to date, alone or in collaboration with other Universities or Research centres, two patents have been filed relative to the production of medicines as protease inhibitors for the treatment of illnesses produced by protozoa of the genus Cryptosporidium (P9701029 Use of maslinic acid as a serine protease inhibitor for the treatment of illnesses caused by parasites of the genus Cryptosporidium). In addition, clinical trials using MDCK cell lines show an infection inhibition percentage of 92.3% at 37 mg/mL. In the case of AIDS-causing viruses, the tests have resulted in a patent (P9702528 Use of maslinic acid as a protease inhibitor for the treatment of the illness caused by the acquired immunodeficiency virus), as it has been found to act intracellularly and considerably inhibits the exit of the virus from the infected cell towards the medium, a mechanism that seems to work with the use of serine proteases. More recently, the Departments of Pharmacology and Organic Chemistry of the University of Granada have carried out a study on hepatoprotection with magnificent results, included in the publication "Antioxidant Activity of Maslinic Acid, a Triterpene obtained from Olea europaea" M.Pilar Montilla, Ahmad Agil, M. Concepción Navarro, M. Isabel Jiménez, Andrés García-Granados, Andrés Parra and Matilde Cabo, Planta Medica 2003, 69, 472-474, which revealed that maslinic acid reduces lipoperoxide levels and membrane hepatocyte susceptibility to lipid peroxidation (LPO), thereby producing a resistance to oxidative stress in rats. On the other hand, researchers from the Universities of Granada and Jaén have performed detailed experiments on rainbow trout, demonstrating that the additivation of specific amounts of maslinic acid to their feed results in a significant improvement of the hepatic organ and function and, therefore, of the animal's health. All of these results have been included in the patent titled by the University of Granada "Maslinic acid as an additive in animal production, P200401679".

As in the case of the aforementioned oleanolic acid, the growing interest in this product is obvious in the large number of patents filed in which maslinic acid acts as an active component: Antitumor agent US20030355201 20030131; Apoptosis inductor (W02002JP13663 20021226); Antiobestic foods and drinks (WO2002JP11608 20021107); External agent for the skin and whitening agent (US20020259323 20020930); Antiobesity drugs and materials thereof (W02002JP07709 20020730); Drugs for vascular lesion (W02002JP03189 20020329); Antitumor food or beverage (W02001 J P 11374 20011225).

Special attention must also be given to another olive component, 3.4-dihydroxyphenyl ethanol (hydroxytyrosol), on which there exist more than 60 patents aimed at protecting highly varied production processes. Thus, researchers from the University of Granada developed in 1992 a "Method of use of alpechin for the production of acids, phenols, alcohols and derivatives by means of counter-flow processes" (Spanish Patent application: 9298236), a process fundamentally aimed at the use of hydroxytyrosol based on alpechin from olives, obtained by means of the three-phase process, although applicable to the aqueous part contained in the "alpeorujo". This process, which consists of the concentration of vegetation water, de-oiled from the extract, subsequent extraction with ethyl acetate and separation of the contents of this extract by means of chromatography-based processes allowed the high-purity isolation of the biophenols present in this extract (fundamentally tyrosol and hydroxytyrosol). Afterwards, the University of Granada developed and patented a process for the production of mannitol from these same derivatives (Use of olive branches and leaves and peduncles and olive alpechin for the production of mannitol and derivatives, P9300490), (Process for the production of mannitol and by-products from the "alpeorujo" resulting from two-phase olive processing, P9300945). On consolidating the milling process to the so-called two-phase process (without added water), new works have arisen aimed at the isolation of hydroxytyrosol: [Production in Large Quantities of Highly Purified Hydroxytyrosol from Liquid-Solid Waste of Two-Phase Olive Oil Processing or "alpeorujo" by Juan Fernández-Bolaños, Guillermo Rodríguez, Rocio Rodríguez, Antonia Heredia, Rafael Guillén, and Ana Jiménez, Agric. Food Chem., 50 (23), 6804 -6811 (2002)], having patented different processes: Method of obtaining a hydroxytyrosol-rich composition from vegetation water (Spanish Patent P200100346) (PCT/ES02/00058). Methods for isolating hydroxytyrosol concentrates from pitted olives have also been developed, treating the vegetation waters with citric acid and subsequently incubating, for the purpose of obtaining the hydroxytyrosol concentrate without solvents (US Patent 6197308 and 6165475).

Hydroxytyrosol concentrates are marketed with the names Hidrox® (Internet Address http://www.creagri.com/hidrox/proprietary.html) and related products "Method of obtaining a hydroxytyrosol-rich composition from vegetation water" (US Patent 6416808) (Internet Address http://www.patentstorm.us/patents/6416808.html). In Spain, these are marketed with the name Hytolive® (Internet Address http://www.hytolive.com) (Internet Address http://www.nutraingredients.com/news/news.asp?id=38632) and in Portugal as Olidrox® (Internet Address http://www.cotecportugal.pt/cotec/images/pdf/Olidrox.pdf).

In addition to the process described in the patents with previous pitting, in the other isolation processes, the line of action consists of a hydrothermal treatment and provoking a prior hydrolysis of various natural components that structurally contain tyrosol and hydroxytyrosol, with or without catalysis, performing filtration processes with membranes and subsequently isolating these phenols in a greater or lesser degree of purity by separation with various types of exchange resins. On the other hand, a large quantity of processes have been developed for the isolation of antioxidants from olive leaves (for example, European Patent EP1389465).

The University of Granada has an industrial patent (P9601652, W098/04331, Process for the industrial use of 3beta-hydroxy-olean-12-en-28-oic (oleanolic) acid and 2alpha,3beta-dihydroxy-olean-12-en-28-oic (maslinic) acid contained in the olive milling by-products), which allows the industrial production of these two acids, separately and in a high degree of purity, from solid by-products of industrial olive milling, by any of the currently used processes (presses, continuous in the three-phase and the so-called two-phase), which represents an attainable and practically inexhaustible source thereof.

In the industrial tuning of this patent, it has been possible to isolate, in an industrially profitable manner, a natural biophenol concentrate by means of processes very different to those described in the aforementioned patents. The process used is efficient and very low-cost, also bearing in mind that it relates to the use of a by-product from the general method used for the production of oleanolic and maslinic acids. For this purpose, the University of Granada has established a new process captured in patent application P200600536 titled "Process for the industrial use of Tyrosol and Hydroxytyrosol contained in the solid by-products of industrial olive milling", due to which it is also available for the production and application of this range of biophenols for the production of functional foods, alone or combined with other natural olive components.

On the other hand, liposomes are spherical vesicles obtained from the interaction of phospholipids. Their formation is not spontaneous but rather induced by the presence of water.

Phospholipids are antipathic molecules, that is, they are comprised of two different parts, one of which is hydrophilic and the other hydrophobic. This characteristic of phospholipids is the cause, when dispersed in water, of the molecules becoming oriented in space, forming micellar structures known as liposomes. Liposomes are characterised by being spherical structures that contain the aqueous medium used for their dispersion.

Liposomes were initially used as cellular membrane models for biophysical studies due to their structural similarity to the cytoplasmatic membranes of most of the cells of living organisms. It was precisely this similarity which gave rise to the idea of their use as a method for transporting substances in topical and parenteral administration.

### DESCRIPTION OF THE INVENTION

For the extraction of the triterpenes present in the composition with liposomes we can use, amongst other processes, that described in a patent developed and titled by the University of Granada (ES211498, Process for the industrial use of 3beta-hydroxy-olean-12-en-28-oic (oleanolic) acid and 2alpha,3beta-dihydroxy-olean-12-en-28-oic (maslinic) acid contained in olive milling by-products) which allows the industrial production of these two acids, separately and in high degree of purity, from solid by-products of industrial olive milling, by any of the currently used processes (presses, continuous in the three-phase and the so-called two-phase), which represents an attainable and practically inexhaustible source thereof. The separation process established is totally physical and extremely effective, allowing the two products to be isolated in the desired proportions from complex original mixtures. This allows us to obtain natural oleanolic and maslinic acid by recovering these from the previously milled olive; due to the extremely hydrophobic nature of these triterpenes, they are immediately associated to the hydrophobic part of the phospholipids, due to which their inclusion is simple and immediate. The amount of substance to be added is limited by the amount of phospholipids present in the liposomic dispersion.

For the extraction of biophenols (fundamentally hydroxytyrosol and tyrosol) present in the composition of liposomes, we can use, amongst other processes, that described in the patent application filed by the University of Granada (P200600536 titled "Process for the industrial use of Tyrosol and Hydroxytyrosol contained in the solid by-products of industrial olive milling"). As in the case of the aforementioned triterpenes, the separation process established is totally physical and extremely effective, allowing us to isolate these products in the desired proportions from the complex original mixtures. This allows us to obtain natural hydroxytyrosol and tyrosol, recovering these from the previously milled olive; due to the hydrophilic nature of the biophenols, they are immediately associated to the hydrophilic part of the biomembranes, due to which their inclusion is simple and immediate.

For the preparation of the formulation in liposomes, take a specific amount of spherical liposomes (not laminated), which are readily available on the market, and add the biophenols dissolved in a minimum amount of water. This hydrophilic composition is added, preferably hot and by stirring vigorously, to a hydrophobic base already containing the triterpenes.

This preparation method accepts from 0.0001% to 20% in weight of final product of the natural biophenols and triterpenes in liposomes.

Thus, we obtain a formulation of biophenols and triterpenes obtained from the genus Olea in liposomes, which is composed of at least one of the triterpenes of the group consisting of maslinic acid and oleanolic acid and/or their salts, and at least one of the biophenols of the group consisting of hydroxytyrosol and/or tyrosol and by liposomes. The biophenols and triterpenes are added to the liposomes as natural products contained in species, subspecies or varieties of the genus Olea that contain at least one of the triterpenes and/or biophenols in an amount greater than 0.001 % of the dry vegetable matter, or the triterpenes and/or biophenols are extracted from the fruit-mill waste of species, subspecies or varieties of the genus Olea that contain at least one of the triterpenes and/or biophenols in an amount greater than 0.001 % of the dry waste.

In addition, we recommend, although it does not limit the invention, that the triterpenes and/or biophenols used are in the form of concentrate or extract of species, subspecies or varieties of the genus Olea, and in solid or liquid form, and that at least one of the selected triterpenes and/or biophenols is present in the amount of between 10% and 95% of the total mixture of triterpenes and/or biophenols respectively.

### Embodiment

One of the methods for preparing a cream with the previously described characteristics is comprised of the following steps:
- Take a mixture of maslinic acid and oleanolic acid and dissolve it in hot propylene glycol.
- Add cetyl alcohol and white wax by stirring and continue to heat the mixture.
- Meanwhile, prepare, also hot, a mixture comprised of distilled water, sodium lauryl sulphate, liposomes, hydroxytyrosol and tyrosol. Stirring vigorously while still hot, add the aqueous phase to the lipid phase, thereby obtaining a white cream suitable for use.
- A more fluid cream can be obtained by slightly increasing the amount of distilled water used.

### Example one:

By way of representative, but not limiting, example of the preparation of a cream, we will describe the addition of triterpenes and biophenols to the world-famous base Beeler, duly liposomed for the production of 102 grams of preparation:
- Take 2 grams of the mixture that contains 85% of maslinic acid and 14% of oleanolic acid and dissolve it in 10 grams of hot propylene glycol; add 15 grams of cetyl alcohol and 1 gram of white wax by stirring and continue to heat the mixture.
- Meanwhile, prepare, also hot, a mixture of distilled water (36 grams), sodium lauryl sulphate (2 grams), liposomes (36 grams) and 0.2 grams of biophenols (90% hydroxytyrosol and 7% tyrosol). Stirring vigorously while still hot, add the aqueous phase to the lipid phase, thereby obtaining a white cream suitable for use.
- A more fluid cream can be obtained by slightly increasing the amount of distilled water used.

In order to prepare a liposomed cream only with biophenols, follow the method described but without including the corresponding triterpenes in the apolar phase.

### Example two:

We can start with a prior propylene glycol base (10 grams) containing between 0.1 and 2 grams of triterpenes and between 0.1 and 2 grams of biophenols. To this base add, hot and by stirring, 15 grams of cetyl alcohol and 1 gram of white wax. Meanwhile, prepare, also hot, a mixture of distilled water (36 grams), sodium lauryl sulphate (2 grams), liposomes (36 grams). Stirring vigorously while still hot, add the aqueous phase to the lipid phase, thereby obtaining a white cream suitable for use.

### Example three:

The formulation of the invention may have the following composition:

| | *mg*/*100 g* |
|---|---|
| Maslinic Acid | 0-20000 |
| Oleanolic Acid | 0-10000 |
| Tyrosol | 1-20000 |
| Hydroxytyrosol | 1-10000 |
| Propylene glycol | 0-50000 |
| Liposomes | 100-99000 |
| Cetyl alcohol | 0-50000 |
| White wax | 0-50000 |
| Sodium lauryl sulphate | 0-50000 |
| Distilled water | q.s. 100 g |

In a specific embodiment, the formulation has the following formulation:

| | *g*/*100 g* |
|---|---|
| Maslinic Acid | 1.7 |
| Oleanolic Acid | 0.28 |
| Tyrosol | 0.18 |
| Hydroxytyrosol | 0.02 |
| Propylene glycol | 10 |
| Liposomes | 36 |
| Cetyl alcohol | 15 |
| White wax | 1 |
| Sodium lauryl sulphate | 2 |
| Distilled water | q.s. 100 g |

## Claims

1. Formulation of natural biophenols obtained from the genus Olea in liposomes, which includes at least one of the biophenols of the group consisting of hydroxytyrosol and tyrosol.

2. Formulation of natural biophenols and triterpenes obtained from the genus Olea in liposomes, which includes at least one of the biophenols of the group consisting of hydroxytyrosol and tyrosol and at least one of the triterpenes of the group consisting of maslinic acid and oleanolic acid and/or their salts.

3. Formulation of natural biophenols in liposomes according to the preceding claims, **characterised in that** the biophenols are added as natural products contained in species, subspecies and varieties of the genus Olea to the liposomes.

4. Formulation of natural biophenols in liposomes according to any of the preceding claims**, characterised in that** the biophenols are extracted from the fruit-mill waste of species, subspecies and varieties of the genus Olea.

5. Formulation of natural biophenols in liposomes according to claims 1 or 2, **characterised in that** the vegetable matter is obtained from species, subspecies or varieties of the genus Olea that contain at least one of the biophenols in an amount greater than 0.001 % of the dry vegetable matter.

6. Formulation of natural biophenols in liposomes according to claim 4, **characterised in that** the fruit mill-waste is obtained from species, subspecies or varieties of the genus Olea that contain at least one of the biophenols in an amount greater than 0.001 % of the dry waste.

7. Formulation of natural biophenols in liposomes according to claims 1 or 2, **characterised in that** at least one of the selected biophenols is present in the amount of between 10% and 95% of the total biophenol mixture used.

8. Formulation of natural biophenols in liposomes according to claims 1 or 2, **characterised in that** said composition contains from 0.0001 % to 20% in weight of natural biophenol in liposomes.

9. Formulation of natural triterpenes and biophenols in liposomes according to claim 2, where said composition contains from 0.0001 % to 20% in weight of natural triterpene and from 0.0001% to 20% of biophenols in liposomes.

10. Formulation of natural triterpenes and biophenols in liposomes according to claim 9, where said composition is obtained from a natural triterpene concentrate containing more than 90% of triterpenes and with a natural biophenol concentrate containing more than 90% of biophenols.

11. Formulation according to claims 1 or 2, **characterised in that** it has the following composition:
| | *mg*/*100 g* |
|---|---|
| Maslinic Acid | 0-20000 |
| Oleanolic Acid | 0-10000 |
| Tyrosol | 1-20000 |
| Hydroxytyrosol | 1-10000 |
| Propylene glycol | 0-50000 |
| Liposomes | 100-99000 |
| Cetyl alcohol | 0-50000 |
| White wax | 0-50000 |
| Sodium lauryl sulphate | 0-50000 |
| Distilled water | q.s. 100 g |

12. Formulation in liposomes, **characterised in that** it has the following composition:
| | *g*/*100 g* |
|---|---|
| Maslinic Acid | 1.7 |
| Oleanolic Acid | 0.28 |
| Tyrosol | 0.18 |
| Hydroxytyrosol | 0.02 |
| Propylene glycol | 10 |
| Liposomes | 36 |
| Cetyl alcohol | 15 |
| White wax | 1 |
| Sodium lauryl sulphate | 2 |
| Distilled water | q.s. 100 g |

13. Formulation in liposomes according to claim 12, **characterised in that** it uses a propylene glycol base or solvent of similar polarity capable of dissolving a triterpene and biophenol concentrate and to which the rest of the components are added.
